(19) Europäisches Patentamt
European Patent Office
Office européen des brevets

(11) Veröffentlichungsnummer : **0 401 613 B1**

# (12) EUROPÄISCHE PATENTSCHRIFT

(45) Veröffentlichungstag der Patentschrift :
**04.01.95 Patentblatt 95/01**

(51) Int. Cl.[6] : **G03C 7/305,** G03C 7/36,
// C07D403/06

(21) Anmeldenummer : **90109915.0**

(22) Anmeldetag : **24.05.90**

## (54) Farbfotografisches Aufzeichungsmaterial mit einem DIR-Kuppler.

(30) Priorität : **06.06.89 DE 3918395**

(43) Veröffentlichungstag der Anmeldung :
**12.12.90 Patentblatt 90/50**

(45) Bekanntmachung des Hinweises auf die Patenterteilung :
**04.01.95 Patentblatt 95/01**

(84) Benannte Vertragsstaaten :
**BE DE FR GB**

(56) Entgegenhaltungen :
**EP-A- 0 267 491**
**EP-A- 0 287 833**
**DE-A- 2 725 892**

(73) Patentinhaber : **Agfa-Gevaert AG**
**Kaiser-Wilhelm-Allee**
**D-51373 Leverkusen (DE)**

(72) Erfinder : **Bergthaller, Peter, Dr.**
**Leuchter Gemark 5 A**
**D-5060 Bergisch Gladbach 2 (DE)**
Erfinder : **Krüger, Thomas, Dr.**
**Friedrich-Engels-Strasse 18**
**D-5090 Leverkusen (DE)**
Erfinder : **Vetter, Hans, Dr.**
**Gerstenkamp 19**
**D-5000 Köln 80 (DE)**
Erfinder : **Odenwälder, Heinrich, Dr.**
**Am Arenzberg 37**
**D-5090 Leverkusen 3 (DE)**

EP 0 401 613 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein farbfotografisches Aufzeichnungsmaterial mit mindestens einer lichtempfindlichen Silberhalogenidemulsionsschicht, die einen Kuppler enthält, der bei Farbentwicklung einen Entwicklungsinhibitor freisetzt.

Es ist bekannt, die chromogene Entwicklung in Gegenwart von Verbindungen durchzuführen, die bei der Entwicklung bildmäßig diffusionsfähige Substanzen freisetzen, die eine bestimmte Wirkung entfalten, beispielsweise die Entwicklung von Silberhalogenid zu beeinflussen vermögen. Falls diese Wirkung darin besteht, daß die weitere Entwicklung inhibiert wird, werden derartige Verbindungen als sogenannte DIR-Verbindungen (DIR = development inhibitor releasing) bezeichnet. Bei den DIR-Verbindungen kann es sich um solche handeln, die unter Abspaltung eines Inhibitorrestes mit dem Oxidationsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet.

DIR-Kuppler sind beispielsweise bekannt aus US-A-3 148 062, US-A-3 227 554, US-A-3 615 506, US-A-3 617 291 und DE-A-24 14 006.

Bei freigesetzten Entwicklungsinhibitoren handelt es sich in der Regel um heterocyclische Mercaptoverbindungen oder um Derivate des Benzotriazols. DIR-Kuppler, die als Entwicklungsinhibitor monocyclische Triazole freisetzen, sind beispielsweise beschrieben in DE-A-28 42 063 und DE-A-0 272 573. Durch Anwendung von DIR-Verbindungen kann eine Vielzahl von fotografischen, die Bildqualität beeinflussenden Effekten bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation "Development-Inhibitor-Releasing (DIR) Couplers in Color Photography" von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, daß sie universell einsetzbar sind, so daß die gleiche Verbindung ohne Rücksicht auf die zu erzeugende Farbe in allen lichtempfindlichen Schichten eines farbfotografischen Aufzeichnungsmaterials verwendet werden kann. DIR-Kuppler können dagegen wegen der aus ihnen erzeugten Farbe meist nur in einem Teil der lichtempfindlichen Schichten verwendet werden, falls nicht die auf sie zurückzuführende Farbnebendichte in den anderen Schichten tolerierbar ist. Diesem Vorteil der DIR-Verbindungen steht als Nachteil gegenüber, daß sie im allgemeinen weniger reaktiv sind als die DIR-Kuppler. In der Praxis hat man sich daher meist darauf beschränkt, DIR-Kuppler zu verwenden und zwar notfalls zwei oder mehrere verschiedene im gleichen Aufzeichnungsmaterial, wobei den unterschiedlich spektral sensibilisierten Schichten verschiedene DIR-Kuppler nach Maßgabe der aus den letzteren erzeugten Farbe zugeordnet werden können.

Es ist normalerweise wichtig, daß der Entwicklungsinhibitor bei Entwicklung rasch aus dem Kuppler freigesetzt wird, weil er den weiteren Verlauf der Entwicklung beeinflussen soll. Es ist daher sehr erwünscht, wenn die betreffenden Kuppler sehr aktiv sind. Hochaktive DIR-Kuppler sind beispielsweise beschrieben in EP-A-0 287 833.

Der Erfindung liegt die Aufgabe zugrunde, ein farbfotografisches Aufzeichnungsmaterial anzugeben, das DIR-Kuppler mit einem an die Kupplungsstelle gebundenen Silberhalogenidentwicklungsinhibitor enthält, aus denen bei der Entwicklung der Inhibitor rasch freigesetzt wird.

Gegenstand der Erfindung ist ein farbfotografisches Aufzeichnungsmaterial mit mindestens einer lichtempfindlichen Silberhalogenidemulsionsschicht und einem dieser zugeordneten DIR-Kuppler, dadurch gekennzeichnet, daß der Kuppler der folgenden Formel I entspricht

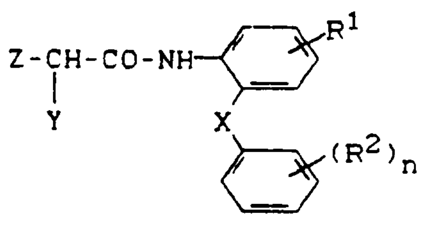

I

worin bedeuten

$R^1$ H, Cl, $-CF_3$, Alkoxy, Sulfamoyl;

$R^2$ Cl, Alkyl, Cycloalkyl, Alkoxy, Alkoxycarbonyl, Carbamoyl;

n 0, 1 oder 2;

X O oder S;
Y einen bei Farbentwicklung freisetzbaren Rest mit Silberhalogenidentwicklungsinhibierungsfunktion;
Z Benzoyl, Pivaloyl, Carbamoyl oder einen Chinazolin-4-on-2-yl-Rest.

Ein in Formel I durch $R^1$ dargestellter Alkoxyrest kann beispielsweise bis zu 16 C-Atome enthalten. Ein durch $R^1$ dargestellter Sulfamoylrest kann ein- oder zweifach substituiert sein, z.B mit Alkyl, Cycloalkyl, Aralkyl oder Aryl; auch können zwei derartige Substituenten zusammen mit dem Stickstoffatom einen 5- oder 6-gliedrigen Ring bilden.

Ein in Formel I durch $R^2$ dargestellter Alkylrest enthält beispielsweise 1 - 10 C-Atome. Ein durch $R^2$ dargestellter Alkoxyrest enthält beispielsweise bis zu 16 C-Atome. Ein durch $R^2$ dargestellter Alkoxycarbonylrest enthält beispielsweise bis zu 17 C-Atome. Für einen durch $R^2$ dargestellten Carbamoylrest gilt das gleiche wie für den unter $R^1$ definierten Sulfamoylrest.

Ein in Formel I durch $R^2$ dargestellter oder in $R^1$ oder $R^2$ enthaltener Cycloalkylrest ist bevorzugt Cyclohexyl.

Ein in Formel I durch Z dargestellter Benzoylrest kann substituiert sein, z.B. mit Alkoxy, vorzugsweise in p-Stellung. Ein in Formel I durch Z dargestellter Carbamoylrest ist bevorzugt von Phenylcarbaminsäure abgeleitet, worin der Phenylring substituiert sein kann, z.B. mit Cl, Alkoxycarbonyl und/oder einer Gruppe

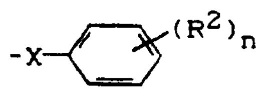

worin X, $R^2$ und n die bereits angegebene Bedeutung haben.

Ein in Formel I durch Z dargestellter Chinazolin-4-on-2-yl-Rest kann substituiert sein, z.B. mit Cl oder Acylamino.

Der in Formel I durch Y dargestellter Rest mit Silberhalogenidentwicklungsinhibierungsfunktion entspricht der Formel

$$-(TIME)_n-INH .$$

Hierin ist INH ein Silberhalogenidentwicklungsinhibitor, n = 0 oder 1, und das durch TIME dargestellte Bindeglied eine Gruppe, die nach Abspaltung aus der Kupplungsstelle des Kupplers bei dessen Kupplung mit dem Oxidationsprodukt des Silberhalogenidentwicklungsmittels befähigt ist, in einer Folgereaktion den daran gebundenen Inhibitor freizusetzen. Die Gruppe TIME wird auch als Zeitsteuerglied bezeichnet, weil bei Anwesenheit einer solchen Gruppe der daran gebundene Inhibitor in vielen Fällen verzögert freigesetzt wird und wirksam werden kann. Bekannte Zeitsteuerglieder sind beispielsweise eine Gruppe

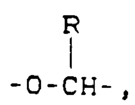

wobei das O-Atom an die Kupplungsstelle des Kupplers und das C-Atom an ein N-Atom eines Inhibitors gebunden ist (z.B DE-A-27 03 145), eine Gruppe, die nach Abspaltung vom Kuppler einer intramolekularen nukleophilen Verdrängungsreaktion unterliegt und hierbei den Inhibitor freisetzt (z.B. DE-A-28 55 697), eine Gruppe, in der nach Abspaltung vom Kuppler eine Elektronenübertragung entlang eines konjugierten Systems stattfinden kann, wodurch der Inhibitor freigesetzt wird (z.B. DE-A-31 05 026), oder eine Gruppe

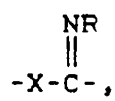

worin X (z.B. -O-) an die Kupplungsstelle des Kupplers und das C-Atom an ein Atom des Inhibitors gebunden ist und worin R beispielsweise für Aryl steht (z.B. EP-A-0 127 063).

Die Gruppe TIME kann vorhanden sein oder auch (im Fall n = 0) völlig fehlen.

DIR-Kuppler der Formel I, bei denen Z für einen Chinazolin-4-on-2-yl-Rest steht, sind bevorzugt und bei diesen wiederum solche, bei denen $R^2$ ein o-Cyclohexylrest ist.

Als Silberhalogenidentwicklungsinhibitoren (Y bzw. INH) werden bevorzugt solche der 1,2,3-Triazol- oder 1,2,4-Triazol-Reihe verwendet.

Beispiele erfindungsgemäßer DIR-Kuppler sind die folgenden.

DIR-1

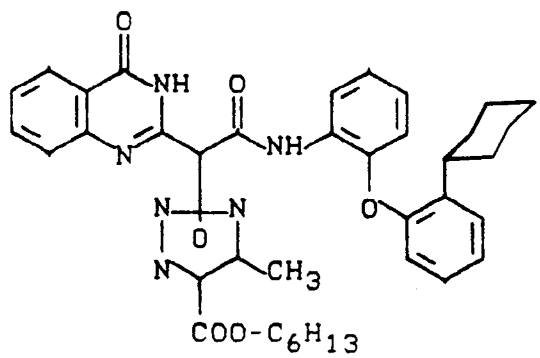

DIR-2

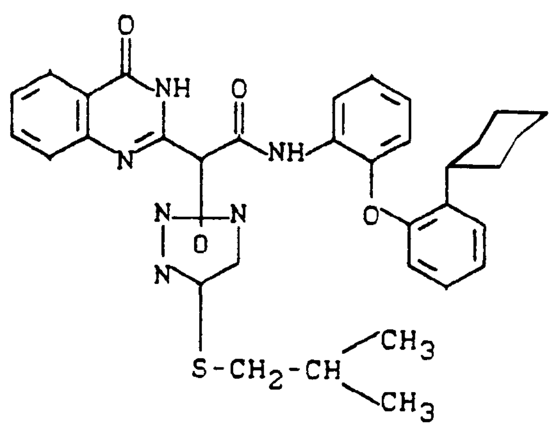

DIR-3

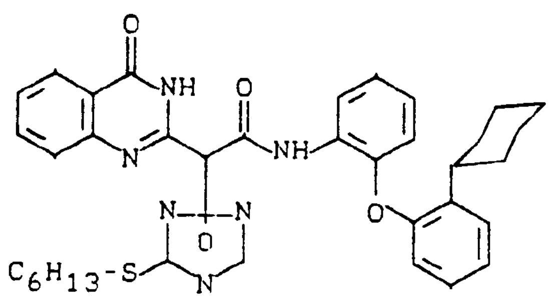

DIR-4

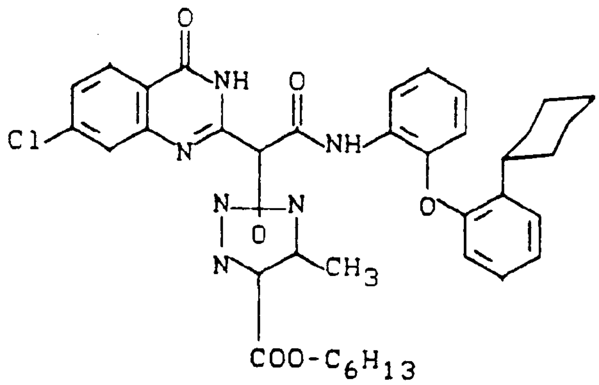

DIR-5

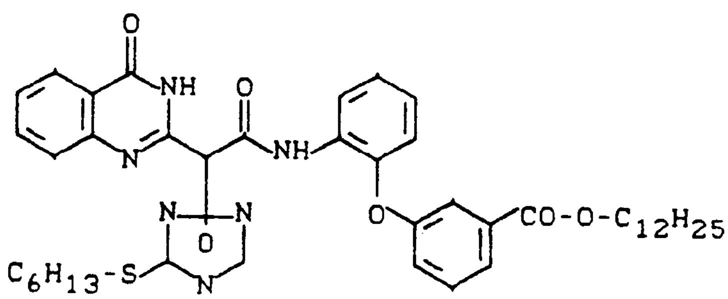

DIR-6

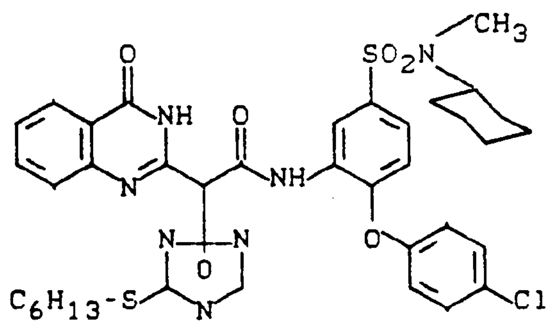

DIR-7

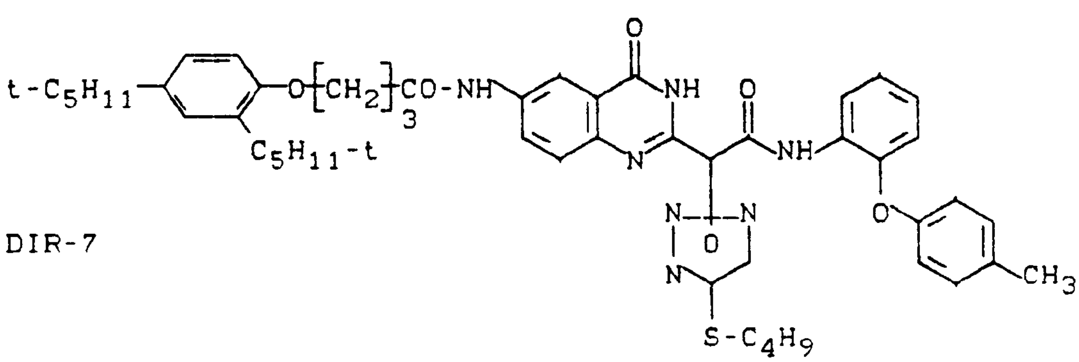

DIR-8

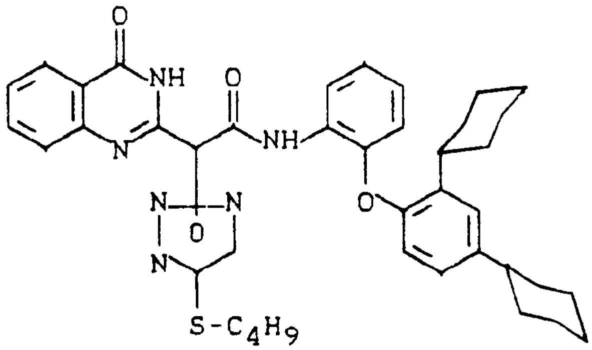

DIR-9

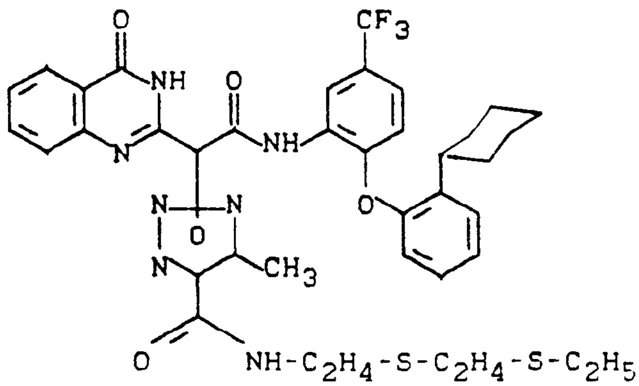

DIR-10

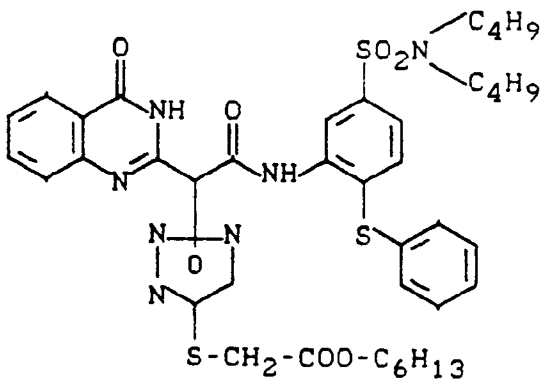

DIR-11

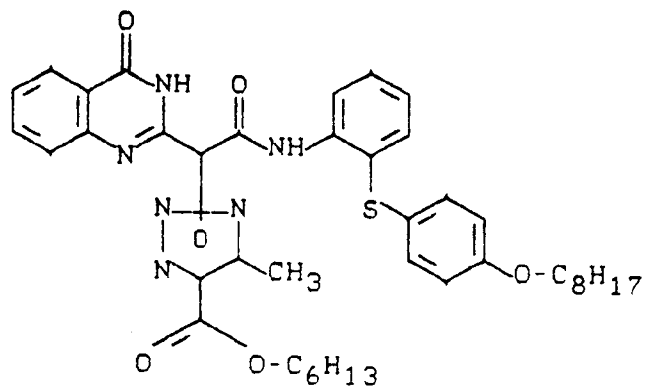

DIR-12

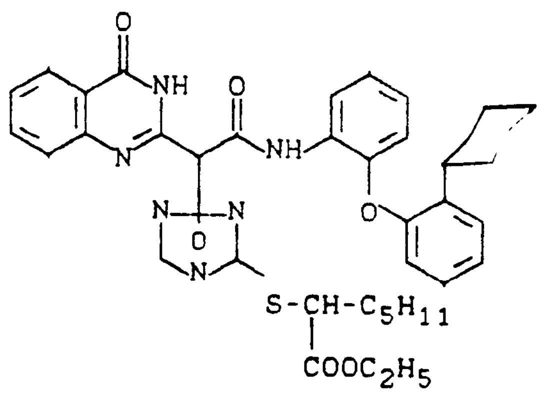

DIR-13

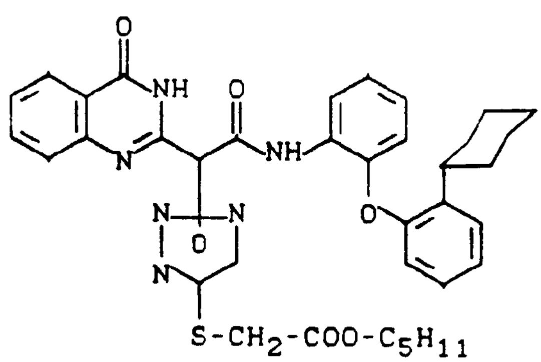

DIR-14

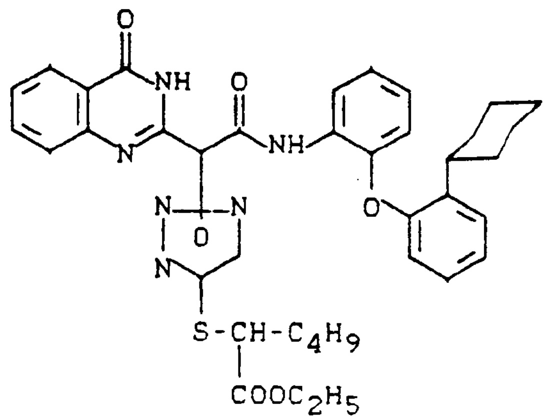

Herstellungsbeispiel 1

DIR-Kuppler DIR-1

1. 2-Cyanacetamido-2′-cyclohexyl-diphenylether

Zu einer Lösung von 45 g Cyanessigsäure (wasserfrei) und 140 g 2-Amino-2′-cyclohexyl-diphenylether, hergestellt durch Umsetzung von 2-Chlornitrobenzol mit 2-Cyclohexyl-phenolkalium in Dimethylsulfoxid und nachfolgende Hydrierung, in 1.000 ml Dichlormethan tropft man bei 30 - 40°C eine Lösung von 103 g Dicyclohexylcarbodiimid in 200 ml Dichlormethan. Man hält anschließend 1 h unter Rückfluß, kühlt auf 20°C und saugt vom ausgeschiedenen Dicyclohexylharnstoff ab. Der Filterrückstand wird mit 400 ml 40°C warmem Dichlormethan nachgewaschen, die vereinigten Filtrate eingedampft, der Rückstand aus Ethylacetat umkristallisiert.
Ausbeute: 240 g (72 % d. Theorie)
Schmelzpunkt: 179-80°C.

2. In eine Suspension von 100 g 2-Cyanacetamino-2′-cyclohexyldiphenylether in 1.000 ml Ethylacetat leitet man nach Zugabe von 23 g Ethanol unter Kühlung auf 0 - 5°C HCl-Gas bis zur Sättigung ein. Man läßt 16 h stehen, engt bei Raumtemperatur im Vakuum auf die Hälfte des Volumens ein, saugt ab und wäscht mit 100 ml Ethylacetat nach.
Ausbeute: 97,5 g (78 % d. Theorie)
Schmelzpunkt: 104°C unter Zersetzung.

Das erhaltene Iminoetherhydrochlorid wird einer auf 85°C erhitzten Lösung von 33 g 2-Aminobenzamid in 140 ml Ethanol unter Rühren portionsweise zugegeben. Man hält noch 2 h unter Rühren am Rückfluß und trägt auf 250 g Eis aus.

Beim Stehen über Nacht tritt Kristallisation ein. Man dekantiert die Mutterlauge ab und kristallisiert aus Acetonitril um:
Ausbeute: 65 g (60 % d. Theorie)
Schmelzpunkt: 197 - 200°C.

3. 3,4-Dihydro-4-oxo-chinazolin-2-bromessigsäure-2-(2-Cyclohexyl)-phenoxyanilid

Eine Suspension von 45,3 g des unter 2. erhaltenen Kupplers wird in 200 ml Essigsäure durch Zugabe von 16 g Brom in 80 ml Essigsäure bei 25 - 30°C bromiert. Man rührt noch 1 h bei 25°C weiter und trägt in 500 ml Eiswasser und 10 g Natriumacetat ein. Nach Stehen über Nacht wird die überstehende Lösung abdekantiert, der Rückstand mit Methanol digeriert und abgesaugt.
Ausbeute: 50 g
Schmelzpunkt: 180°C (Zersetzung)

4. Zu einer Lösung von 10,65 g des bromierten Kupplers aus 3. und 4,5 g 5-Methyl-1,2,3-triazolcarbonsäure-n-hexylester in 150 ml Ethylacetat trägt man unter Rühren 6 g Kaliumcarbonat ein. Man rührt insgesamt 4 h bei 25 - 30°C, gießt auf 400 ml Wasser und 5 ml Essigsäure, trennt die Ethylacetatphase ab, trocknet mit Natriumsulfat und dampft im Vakuum ein. Das erhaltene rotbraune Öl kristallisiert beim Verrühren mit Ethanol über 24 h aus.
Ausbeute: 8,4 g
Schmelzpunkt: 176 - 178°C

Die Verbindung besteht aus zwei Isomeren, die Hauptmenge besteht aus einem weniger polaren Isomer mit Fp. 172 - 173°C, die Nebenmenge aus einem hochschmelzenden Isomer (Fp. 217°C).

Die DIR-Kuppler der vorliegenden Erfindung sind übereinstimmend durch eine ortho-Aryloxy- bzw. ortho-Arylthio-Substitution im Ballastanilidteil gekennzeichnet. Es handelt sich hierbei um hochwirksame DIR-Kuppler, wobei insbesondere die entsprechenden DIR-Kuppler aus der Chinazolonacetanilidreihe gegenüber allen anderen geprüften Ballastanilidstrukturen erhöhte Reaktivität und damit stärkere Inhibierung, höhere Interimageeffekte und Kanteneffekte zeigen. Dies wirkt sich in einer Verbesserung von Farbwiedergabe und Schärfe, gegebenenfalls auch in verbesserter Körnigkeit, aus.

Die Verbindungen der vorliegenden Erfindung eignen sich für die Verwendung als DIR-Kuppler in farbfotografischen, insbesondere mehrschichtigen Aufzeichnungsmaterialien. Wenn es sich um Gelbkuppler handelt, werden sie bevorzugt in oder zugeordnet zu einer lichtempfindlichen Silberhalogenidemulsionsschicht mit einer überwiegenden Empfindlichkeit für den blauen Spektralbereich des sichtbaren Lichtes verwendet. Der besondere Vorteil der erfindungsgemäßen Kuppler, nämlich eine vergleichsweise geringe Entwicklungsinhibierung in der Schicht, der eine solche Verbindung zugeordnet ist, neben einer vergleichsweise hohen Entwicklungsinhibierung in benachbarten nicht zugeordneten Schichten, kommt naturgemäß besonders dann zum Tragen, wenn es sich um ein mehrschichtiges farbfotografisches Aufzeichnungsmaterial handelt, das neben einer überwiegend blauempfindlichen Silberhalogenidemulsionsschicht weitere lichtempfindliche Silberhalogenidemulsionsschichten enthält mit überwiegender Empfindlichkeit für den grünen bzw. roten Spektralbereich des sichtbaren Lichtes. Kuppler, die nur wenig Farbe bei der Entwicklung ergeben, können wahlweise einer blauempfindlichen, einer grünempfindlichen oder einer rotempfindlichen Schicht oder auch mehreren dieser Schichten zugeordnet werden, ohne daß eine Farbverfälschung zu befürchten ist.

Auch als Farbkuppler können die erfindungsgemäßen DIR-Kuppler wegen ihrer außerordentlich hohen Wirksamkeit in vergleichsweise geringen Mengen eingesetzt werden um die erwünschten Effekte, insbesondere die Interimageeffekte hervorzubringen. Dies ermöglicht es beispielsweise, einen erfindungsgemäßen Gelb-DIR-Kuppler nicht nur in den blauempfindlichen Gelbfarbstoff erzeugenden Schichten sondern auch in anderen Schichten einzusetzen, ohne daß dort eine zu hohe unerwünschte Nebendichte auftritt. Die erfindungsgemäßen DIR-Kuppler sind somit als Gelbkuppler mit Vorteil auch in Purpurschichten wie auch in Blaugrünschichten anwendbar.

Bei der Herstellung des lichtempfindlichen farbfotografischen Aufzeichnungsmaterials können die diffusionsfesten DIR-Kuppler der vorliegenden Erfindung gegebenenfalls zusammen mit anderen Kupplern in bekannter Weise in die Gießlösung der Silberhalogenidemulsionsschichten oder anderer Kolloidschichten eingearbeitet werden. Beispielsweise können öllösliche oder hydrophobe Kuppler vorzugsweise aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemischen davon, evtl. mit einem Gehalt an Silberiodid bis zu 20 mol-% in einem der üblicherweise verwendeten hydrophilen Bindmittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch und spektral sensibilisiert sein, und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht der drei Spektralbereiche Rot, Grün und Blaue. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-25 30 645, DE-A-26 22 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Blaugrün, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (z.B. Blaugrün, Purpur, Gelb) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Bei bevorzugten Ausführungsformen sind rotempfindlichen Silberhalogenidemulsionsschichten mindestens ein nichtdiffundierender Farbkuppler zur Erzeugung des blaugrünen Teilfarbenbildes, grünempfindlichen Silberhalogenidemulsionsschichten mindestens ein nichtdiffundierender Farbkuppler zur Erzeugung des purpurnen Teilfarbenbildes und blauempfindlichen Silberhalogenidemulsionsschichten mindestens ein nichtdiffundierender Farbkuppler zur Erzeugung des gelben Teilfarbenbildes zugeordnet.

Es sind aber auch andere Zuordnungen bekannt.

Farbkuppler zur Erzeugung des blaugrünen Teilfarbenbildes sind in der Regel Kuppler vom Phenol- oder α-Naphtholtyp; geeignete Beispiele hierfür sind

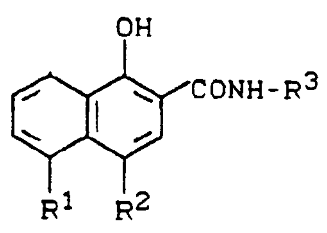

C-1: $R^1$, $R^2$ = H;

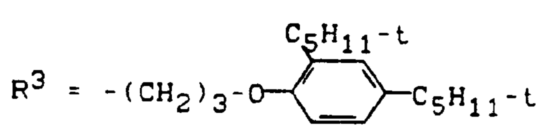

C-2: $R^1 = -NHCOOCH_2-CH(CH_3)_2$; $R^2$ = H; $R^3 = -(CH_2)_3-OC_{12}H_{25}$

C-3: $R^1$ = H; $R^2 = -OCH_2-CH_2-SO_2CH_3$; $R^3 = -C_{16}H_{33}$

C-4: $R^1 = H$; $R^2 = -OCH_2-CONH-(CH_2)_2-OCH_3$;

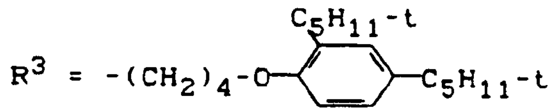

C-5: $R^1$, $R^2 = H$;

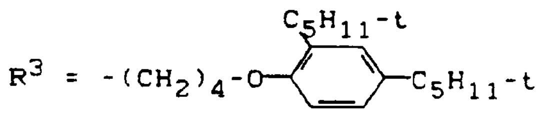

C-6: $R^1$, $R^2 = H$;

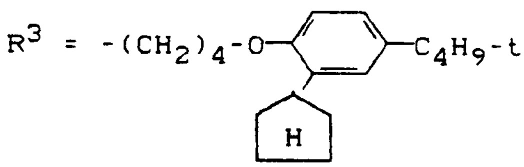

C-7: $R^1 = H$; $R^2 = Cl$; $R^3 = -C(C_2H_5)_2-C_{21}H_{43}$

C-8: $R^1 = H$; $R^2 = -O-CH_2-CH_2-S-CH(COOH)-C_{12}H_{25}$ $R^3$ = Cyclohexyl

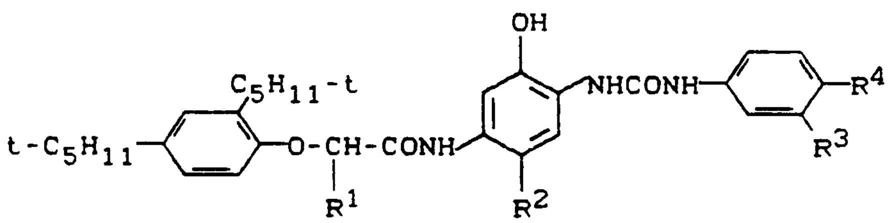

C-9: $R^1 = -C_4H_9$; $R^2 = H$; $R^3 = -CN$; $R^4 = Cl$

C-10: $R^1 = -C_4H_9$; $R^2 = H$; $R^3 = H$; $R^4 = -SO_2CHF_2$

C-11: $R^1 = -C_4H_9$;

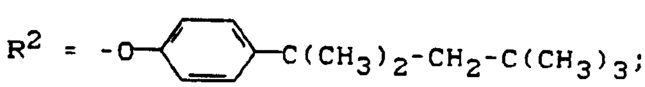

$R^3 = H$; $R^4 = -CN$

C-12: $R^1 = C_2H_5$; $R^2$, $R^3 = H$; $R^4 = -SO_2CH_3$

C-13: $R^1 = -C_4H_9$; $R^2$, $R^3 = H$; $R^4 = -SO_2-C_4H_9$

C-14: $R^1 = -C_4H_9$; $R^2 = H$; $R^3 = -CN$; $R^4 = -CN$

C-15: $R^1 = -C_4H_9$; $R^2$, $R^3 = H$; $R^4 = -SO_2-CH_2-CHF_2$

C-16: $R^1 = -C_2H_5$; $R^2$, $R^3 = H$; $R^4 = -SO_2CH_2-CHF-C_3H_7$

C-17: $R^1 = -C_4H_9$; $R^2$, $R^3 = H$; $R^4 = F$

C-18: $R^1 = -C_4H_9$; $R^2$, $R^3 =H$; $R^4 = -SO_2CH_3$

C-19: $R^1 = -C_4H_9$; $R^2$, $R^3 =H$; $R^4 = -CN$

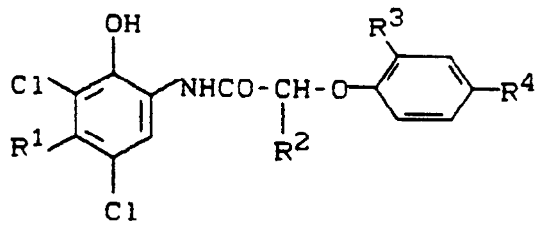

C-20: $R^1$ = $-CH_3$; $R^2$ = $-C_2H_5$; $R^3$, $R^4$ = $-C_5H_{11}$-t
C-21: $R^1$ = $-CH_3$; $R^2$ = H; $R^3$, $R^4$ = $-C_5H_{11}$-t
C-22: $R^1$, $R^2$ = $-C_2H_5$; $R^3$, $R^4$ = $-C_5H_{11}$-t
C-23: $R^1$ = $-C_2H_5$; $R^2$ = $-C_4H_9$; $R^3$, $R^4$ = $-C_5H_{11}$-t
C-24: $R^1$ = $-C_2H_5$; $R^2$ = $-C_4H_9$; $R^3$, $R^4$ = $C_4H_9$-t

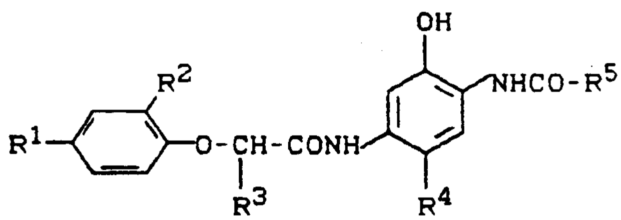

C-25: $R^1$, $R^2$ = $-C_5H_{11}$-t; $R^3$ = $-C_4H_9$; $R^4$ = H; $R^5$ = $-C_3F_7$
C-26: $R^1$ = $-NHSO_2-C_4H_9$; $R^2$ = H; $R^3$ = $-C_{12}H_{25}$; $R_4$ = Cl; $R^5$ = Phenyl
C-27: $R^1$, $R^2$ = $-C_5H_{11}$-t; $R^2$ = Cl, $R^3$ = $-C_3H_7$-i; $R^4$ = Cl; $R^5$ = Pentafluorphenyl
C-28: $R^1$ = $-C_5H_{11}$-t; $R^2$ = Cl; $R^3$ = $-C_6H_{13}$; $R^4$ = Cl; $R^5$ = -2-Chlorphenyl

Farbkuppler zur Erzeugung des purpurnen Teilfarbenbildes sind in der Regel Kuppler vom Typ des 5-Pyrazolons, des Indazolons oder der Pyrazoloazole; geeignete Beispiele hierfür sind

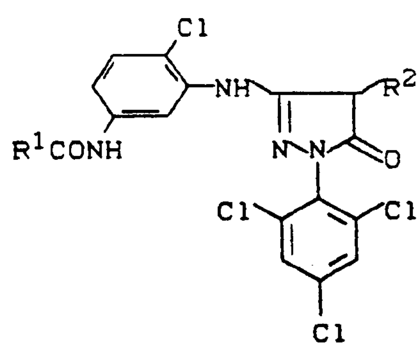

M-1:

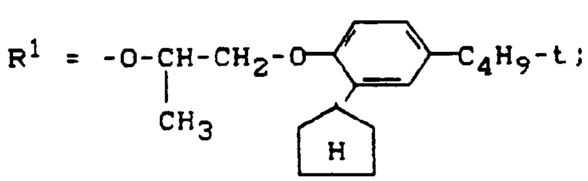

M-2:

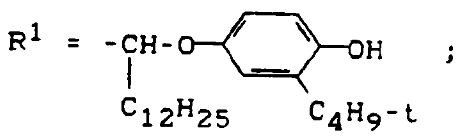

$R^2 = H$

M-3: $R^1 = -C_{13}H_{27}$; $R^2 = H$

M-4: $R^1 = -OC_{16}H_{33}$; $R^2 = H$

M-5: $R^1 = -C_{13}H_{27}$;

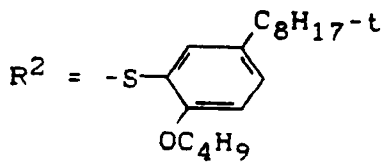

M-6:

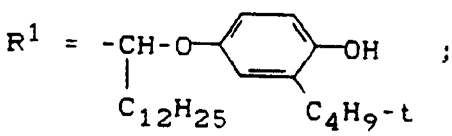

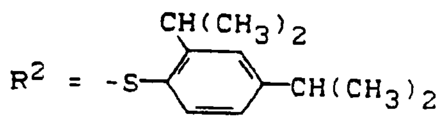

M-7: $R^1 = C_9H_{19}$;

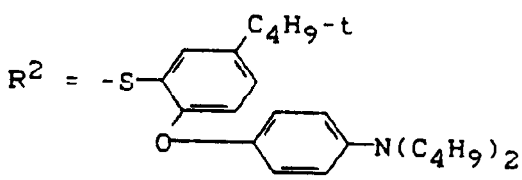

M-8:

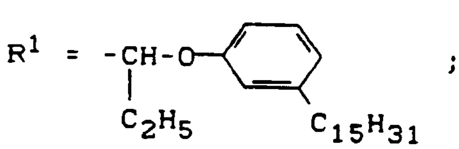

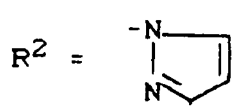

M-9:

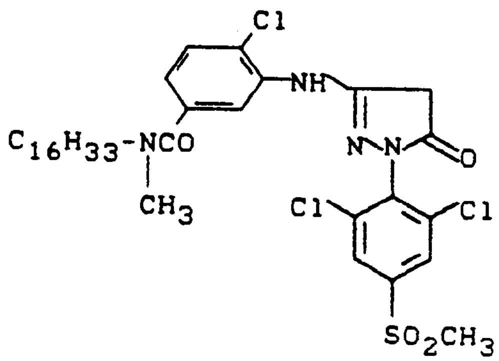

M-10:

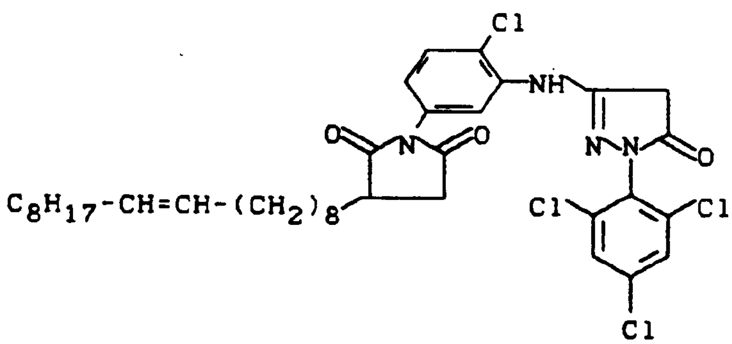

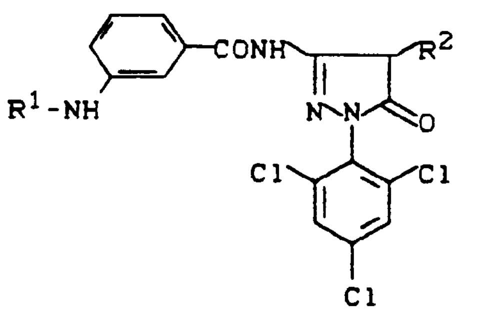

M-11:

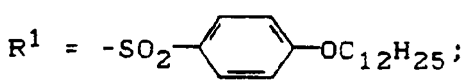

$R^2 = H$

M-12:

$R^1$ = 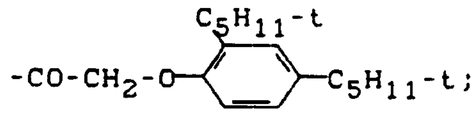

$R^2 = H$

M-13:

$R^1$ = 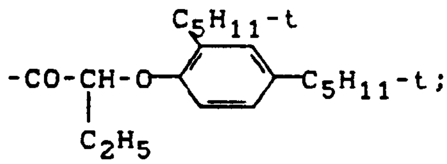

$R^2 = H$

M-14:

$R^1$ = 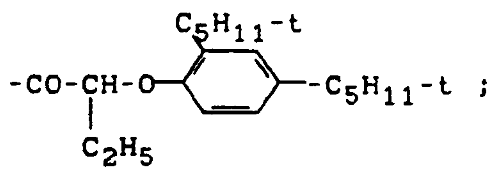

$R^2$ = 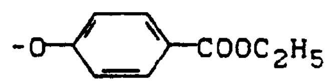

M-15:

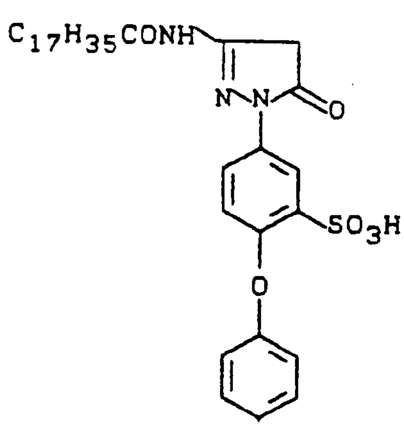

M-16:

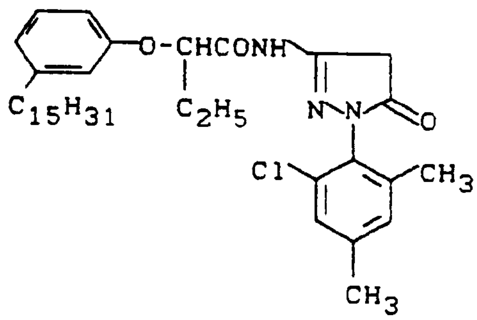

M-17:

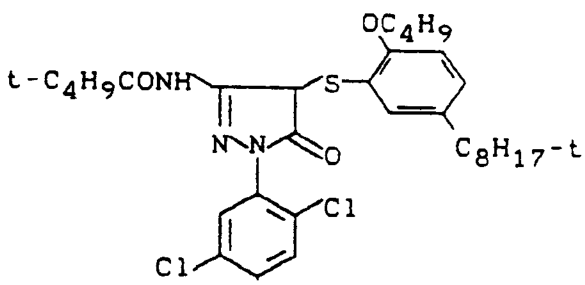

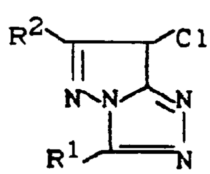

M-18:

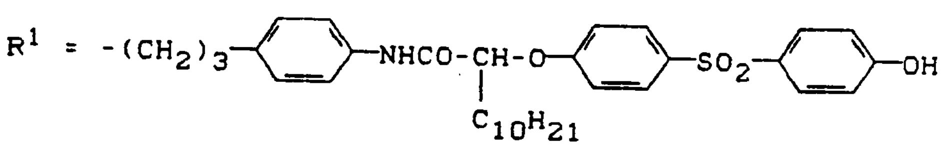

$R^2$ = $-CH_3$

M-19:

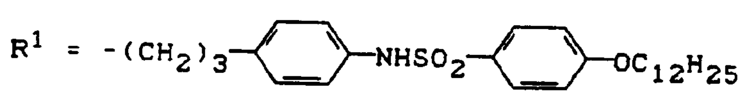

$R^2$ = $-CH_3$

M-20:

$R^1$ = 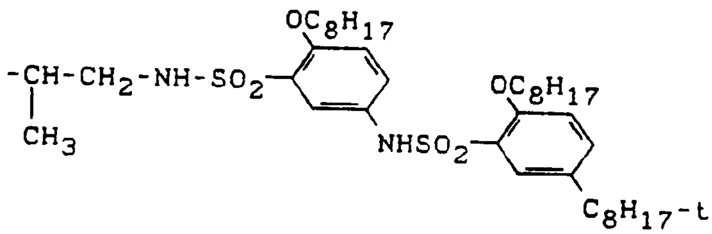

$R^2 = -C_4H_9$-t

M-21:

$R^1$ = 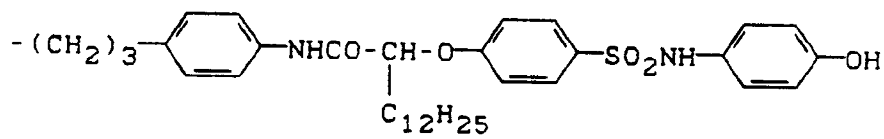

$R^2 = -CH_3$

M-22:

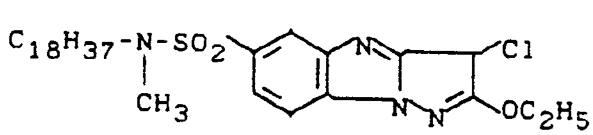

Farbkuppler zur Erzeugung des gelben Teilfarbenbildes sind in der Regel Kuppler mit einer offenkettigen Ketomethylengruppierung, insbesondere Kuppler vom Typ des α-Acylacetamids; geeignete Beispiele hierfür sind α-Benzoylacetanilidkuppler und α-Pivaloylacetanilidkuppler der Formeln

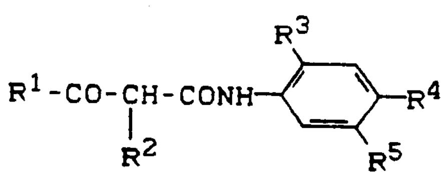

Y-1: $R^1 = -C_4H_9$-t;

$R^2$ = 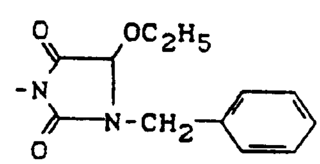 ;

$R^3$ = Cl; $R^4$ = H;

$R^5$ = 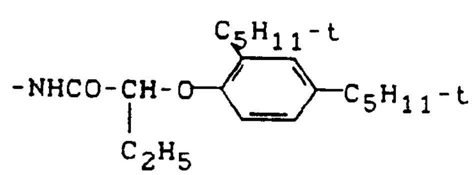

Y-2: $R^1$ = $-C_4H_9$-t;

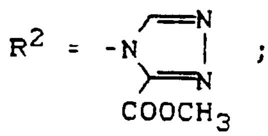

$R^3$ = $-OC_{16}H_{33}$; $R^4$ = H;
$R^5$ = $-SO_2NHCH_3$

Y-3: $R^1$ = $-C_4H_9$-t;

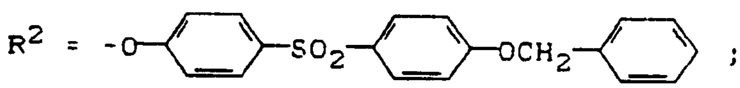

$R^3$ = Cl
$R^4$ = H; $R^5$ = $-NHSO_2-C_{16}H_{33}$

Y-4: $R^1$ = $-C_4H_9$-t;

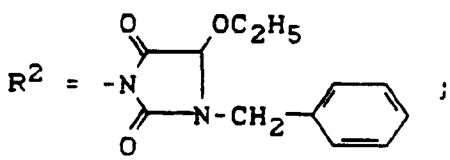

$R^3$ = Cl;
$R^4$ = H; $R^5$ = $-COOC_{12}H_{25}$

Y-5: $R^1$ = $-C_4H_9$-t;

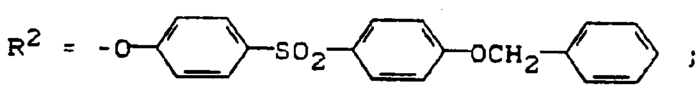

$R^3$ = Cl;
$R^4$ = H;

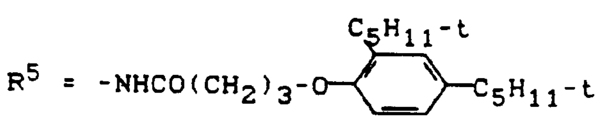

Y-6: $R^1$ = $-C_4H_9$-t;

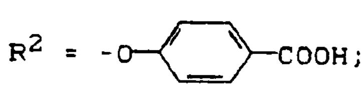

$R^3$ = Cl; $R^4$ = H;

$R^5$ = 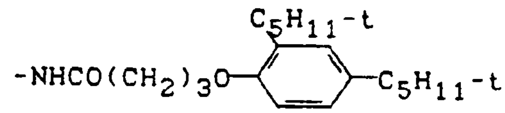

Y-7: $R^1 = -C_4H_9$-t;

$R^2$ = 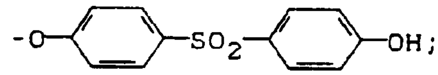

$R^3$ = Cl;
$R^4$ = H; $R^5 = -NHSO_2-C_{16}H_{33}$

Y-8: $R^1 = -C_4H_9$-t;

$R^2$ = 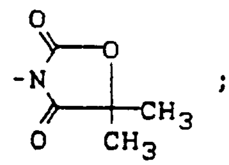

$R^3$ = Cl; $R^4$ = H;

$R^5$ = 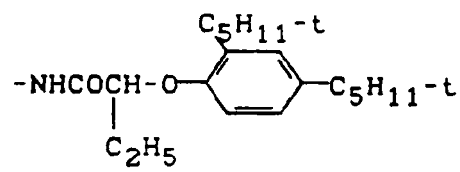

Y-9: $R^1 = -C_4H_9$-t;

$R^2$ = 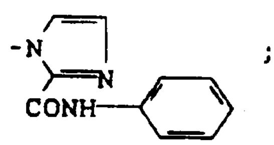

$R^3 = -OC_{16}H_{33}$;
$R^4$ = H; $R^5 = -SO_2NHCOC_2H_5$

Y-10: $R^1 = -C_4H_9$-t;

$R^2$ = 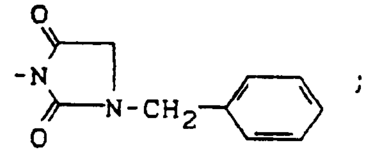

$R^3$ = Cl; $R^4$ = H

$R_3$ = 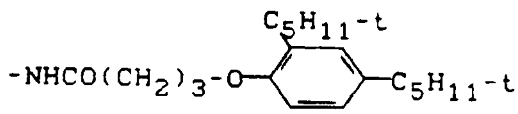

Y-11: $R^1$ = $-C_4H_9$-t;

$R^2$ = 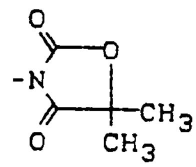 ;

$R^3$ = Cl; $R^4$ = H;

$R^5$ = 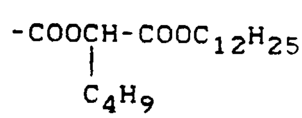

Y-12: $R^1$ = $-C_4H_9$-t;

$R^2$ = 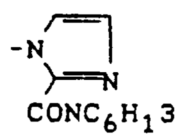 ;

$R^3$ =Cl; $R^4$ = H;

$R^5$ = 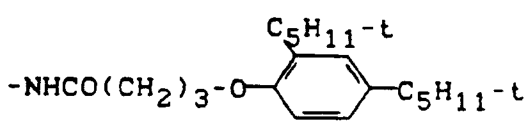

Y-13: $R^1$ = $-C_4H_9$-t;

$R^2$ = 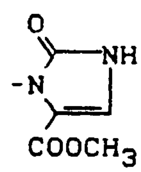 ;

$R^3$ = $-OC_{16}H_{33}$; $R^4$ = H;
$R^5$ = $-SO_2NHCH_3$

$R_3$ = 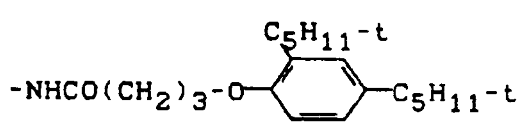

Y-14: $R^1$ = $-C_4H_9$-t;

$R^2$ = 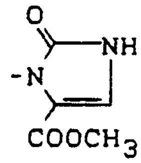 ;

$R^3$ = Cl; $R^4$ = H;

$R^5$ = 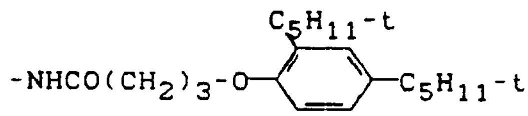

Y-15:

$R^1$ = 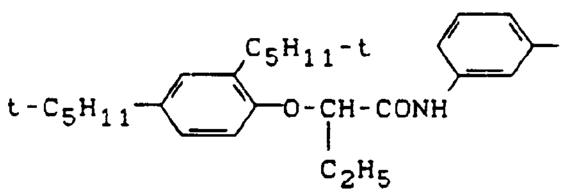 ;

$R^2$, $R^4$, $R^5$ = H; $R^3$ = $-OCH_3$

Y-16:

$R^1$ = 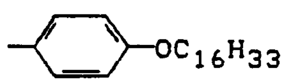 ; $R^2$ = 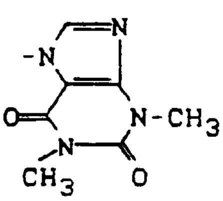

$R^3$, $R^5$ = $-OCH_3$; $R^4$ = H

Y-17:

$R^1$ = 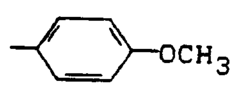 ; $R^2$ = 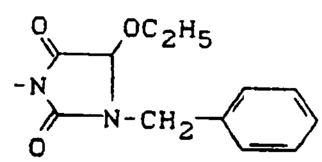

$R^3$ = Cl, $R^4$ = H; $R^5$ = $-COOC_{12}H_{25}$

Y-18:

$R^1$ = 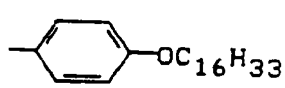 ; $R^2$ = 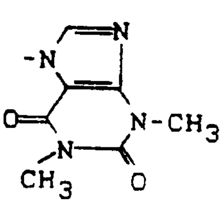

$R^3$ = Cl; $R^4$, $R^5$ = $-OCH_3$

Y-19:

$R^1$ = 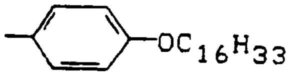 ; $R^2$ = 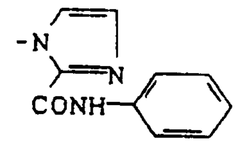

$R^3$ = $-OCH_3$; $R^4$ = H; $R^5$ = $-SO_2N(CH_3)_2$

Y-20:

$R^1$ = 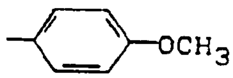 ;

$R^2$ = 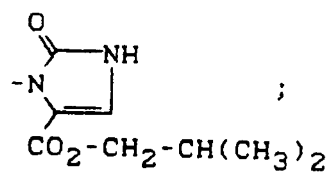 ;

$R^3$ = $-OCH_3$; $R^4$ = H;

$R_5$ = 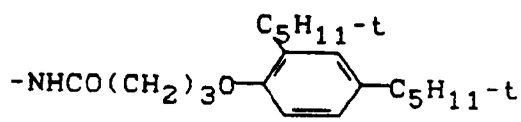

Y-21:

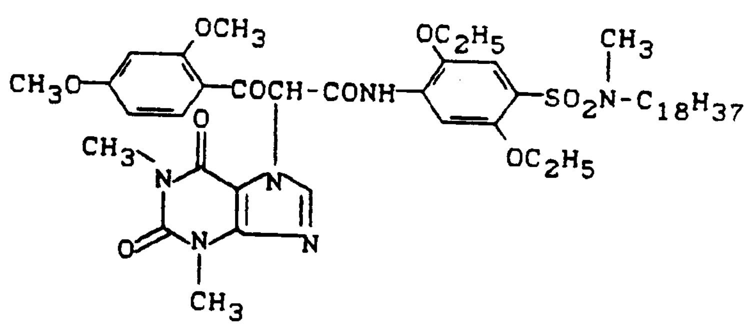

Bei den Farbkupplern kann es sich um 4-Äquivalentkuppler, aber auch um 2-Äquivalentkuppler handeln. Letztere leiten sich von den 4-Äquivalentkupplern dadurch ab, daß sie in der Kupplungsstelle einen Substituenten enthalten, der bei der Kupplung abgespalten wird. Zu den 2-Äquivalentkupplern sind solche zu rechnen, die farblos sind, als auch solche, die eine intensive Eigenfarbe aufweisen, die bei der Farbkupplung verschwindet bzw. durch die Farbe des erzeugten Bildfarbstoffes ersetzt wird (Maskenkuppler), und die Weißkuppler, die bei Reaktion mit Farbentwickleroxidationsprodukten im wesentlichen farblose Produkte ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird und dabei entweder direkt oder, nachdem aus dem primär abgespaltenen Rest eine oder mehrere weitere Gruppen abgespalten worden sind (z.B. DE-A-27 03 145, DE-A-28 55 697, DE-A-31 05 026, DE-A-33 19 428), eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR-bzw. FAR-Kuppler.

Da bei den DIR-, DAR- bzw. FAR-Kupplern hauptsächlich die Wirksamkeit des bei der Kupplung freigesetzten Restes erwünscht ist und es weniger auf die farbbildenden Eigenschaften dieser Kuppler ankommt, kommen auch solche DIR-, DAR- bzw. FAR-Kuppler in Frage, die bei der Kupplung im wesentlichen farblose Produkte ergeben (DE-A-15 47 640).

Der abspaltbare Rest kann auch ein Ballastrest sein, so daß bei der Reaktion mit Farbentwickleroxidationsprodukten Kupplungsprodukte erhalten werden, die diffusionsfähig sind oder zumindest eine schwache bzw. eingeschränkte Beweglichkeit aufweisen (US-A-4 420 556).

Erfindungsgemäß enthält das farbfotografische Aufzeichnungsmaterial zusätzlich mindestens einen DIR-Kuppler der Formel I, wobei dieser Kuppler nicht nur in der Gelbschicht, sondern auch in der Purpurschicht und/oder auch in der Blaugrünschicht sowie auch in einer zu einer der genannten Schichten benachbarten nicht lichtempfindlichen Schicht enthalten sein kann.

Über die genannten Bestandteile hinaus kann das farbfotografische Aufzeichnungsmaterial der vorliegenden Erfindung weitere Zusätze enthalten, zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es vorteilhaft, in einer oder mehreren der in dem Aufzeichnungsmaterial enthaltenen Schichten, vorzugsweise in einer der oberen Schichten, UV-absorbierende Verbindungen zu verwenden. Geeignete UV-Absorber sind beispielsweise in US-A-3 253 921, DE-C-2 036 719 und EP-A-0 057 160 beschrieben.

Für die erfindungsgemäßen Materialien können die üblichen Schichtträger verwendet werden, siehe Research Disclosure Nr. 17 643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Begußhilfsmittel und Weichmacher können verwendet werden. Verwiesen wird auf die in der Research Disclosure Nr. 17 643 in Abschnitt IX, XI und XII angegebenen Verbindungen.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der DE-A-22 18 009 zu härten, um farbfotografische Materialien zu erzielen, die für eine Hochtemperaturverarbeitung geeignet sind. Ferner ist es möglich, die fotografischen Schichten mit Härtern der Diazin-, Triazin- oder 1,2-Dihydrochinolin-Reihe zu härten oder mit Härtern vom Vinylsulfon-Typ. Weitere geeignete Härtungsmittel sind aus den DE-A-24 39 551, DE-A-22 25 230, DE-A-23 17 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Zur Herstellung farbfotografischer Bilder wird das erfindungsgemäße farbfotografische Aufzeichnungsmaterial mit einer Farbentwicklerverbindung entwickelt. Als Farbentwicklerverbindung lassen sich sämtliche Entwicklerverbindungen verwenden, die die Fähigkeit haben, in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer Chem. Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

## Beispiel 1

Ein farbfotografisches Aufzeichnungsmaterial für die Colornegativfarbentwicklung wurde hergestellt

(Schichtaufbau 1 A - Vergleich), indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,1 g 4-Hydroxy-6-methyl-1,3,3a,7-tetraazainden stabilisiert.

Schichtaufbau 1 A (Vergleich)

| | | |
|---|---|---|
| Schicht 1 | (Antihaloschicht) | |
| | schwarzes kolloidales Silbersol mit | |
| | 0,2 g Ag | |
| | 1,2 g Gelatine | |
| | 0,1 g UV-Absorber UV-1 | |
| | 0,2 g UV-Absorber UV-2 | |
| | 0,02 g Trikresylphosphat (TKP) | |
| | 0,03 g Dibutylphthalat (DBP) | |
| Schicht 2 | (Mikrat-Zwischenschicht) | |
| | Mikrat-Silberbromidiodidemulsion | |
| | (0,5 mol-% Iodid; | |
| | mittlerer Korndurchmesser 0,07 µm) | |
| | aus | |
| | | 0,25 g $AgNO_3$, mit |
| | | 1,0 g Gelatine |
| Schicht 3 | (rotsensibilisierte Schicht, mittelempfindlich) | |
| | rotsensibilisierte Silberbromidiodidemulsion | |
| | (4,0 mol-% Iodid; | |
| | mittlerer Korndurchmesser 0,45 µm) | |
| | aus | |
| | | 5,35 g $AgNO_3$, mit |
| | | 3,75 g Gelatine |
| | | 1,33 g Cyankuppler C-19 |
| | | 0,05 g Rotmaske RM-1 |
| | | 0,118 g DIR-Kuppler DIR-A |
| | | 1,33 g TKP |
| | | 0,236 g DBP |
| Schicht 4 | (Zwischenschicht) | |
| | aus | |
| | | 1,43 g Gelatine |
| | | 0,74 g Scavenger |
| Schicht 5 | (grünsensibilisierte Schicht, mittelempfindlich) | |
| | grünsensibilisierte Silberbromidiodidemulsion | |
| | (4,0 mol-% Iodid; | |
| | mittlerer Korndurchmesser 0,45 µm) | |
| | aus | |
| | | 3,10 g $AgNO_3$, mit |
| | | 2,33 g Gelatine |
| | | 0,775 g Magentakuppler M-12 |
| | | 0,050 g Gelbmaske YM-1 |
| | | 0,068 g DIR-Kuppler-DIR-A |
| | | 0,775 g TKP |
| | | 0,136 g DBP |
| Schicht 6 | (Zwischenschicht) | |
| | wie Schicht 4 | |
| Schicht 7 | (Gelbfilterschicht) | |
| | gelbes kolloidales Silbersol | |
| | mit | |
| | | 0,09 g Ag, |
| | | 0,34 g Gelatine |

| | |
|---|---|
| Schicht 8 | (blauempfindliche Schicht, mittelempfindlich), blausensibilisierte Silberbromidiodidemulsion (4,0 mol-% Iodid; mittlerer Korndurchmesser 0,45 µm) ) aus |
| | 3,46 g $AgNO_3$, mit |
| | 1,73 g Gelatine |
| | 1,25 g Gelbkuppler Y-20 |
| | 0,076 g DIR-Kuppler DIR-A |
| | 1,25 g TKP |
| | 0,152 g DBP |
| Schicht 9 | (Zwischenschicht) wie Schicht 4 |
| Schicht 10 | (Schutz- und Härtungsschicht) aus |
| | 0,68 g Gelatine |
| | 0,73 g Härtungsmittel (CAS Reg. No 65411-60-1) |
| | 0,50 g Formaldehydfänger FF |

In Beispiel 1 werden außer den bereits erwähnten Kupplern folgende Verbindungen verwendet:

UV-Absorber UV-1

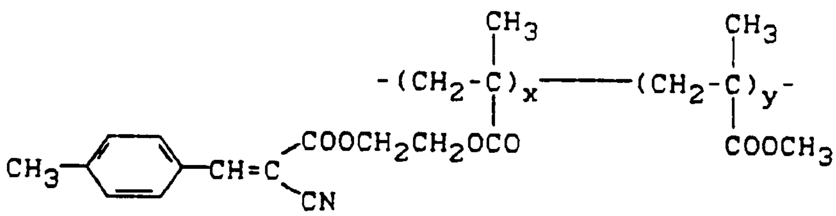

Gewichtsverhältnis: x : y = 7:3

UV-Absorber UV-2

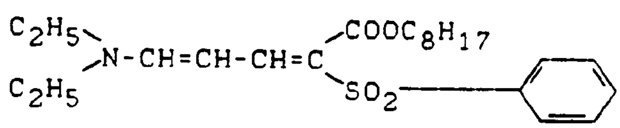

Rotmaske RM-1

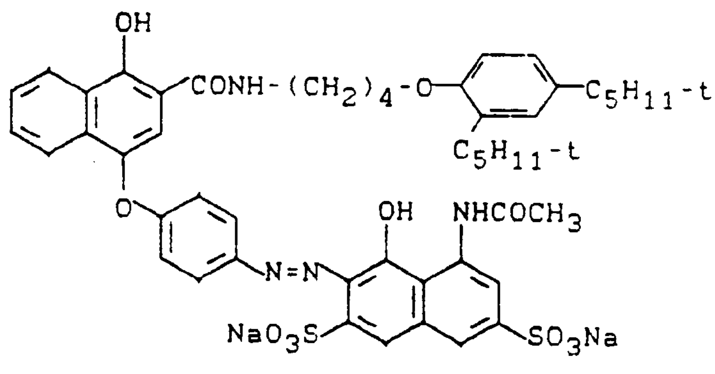

Gelbmaske YM-1

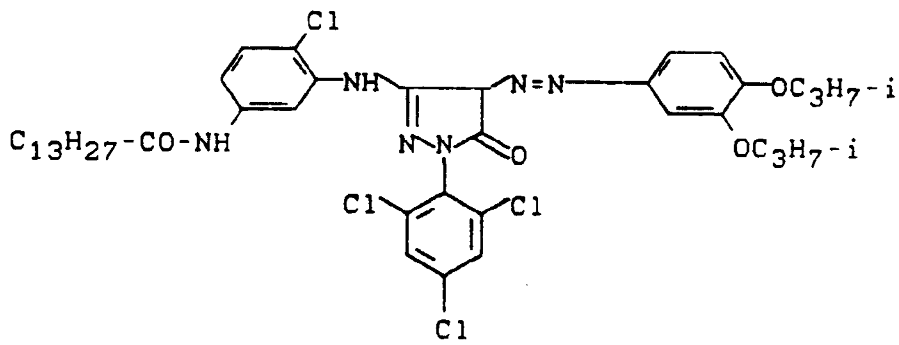

Scavenger SC-1

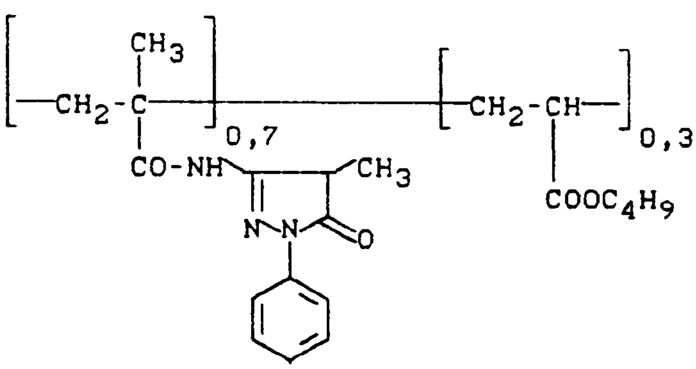

Formaldehydfänger FF

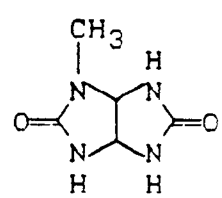

Als Netzmittel ist in allen Schichten Na-perfluorbutansulfonat eingesetzt. In Schichtaufbau 1 A verwendeter DIR-Kuppler (Vergleich) :

DIR-A

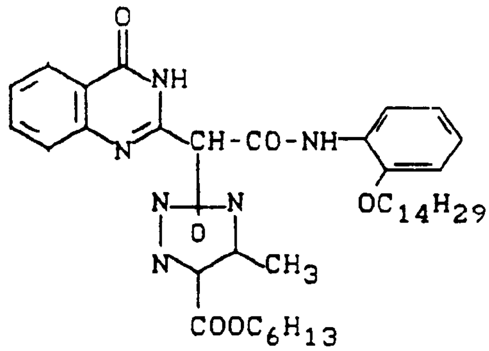

(DIR-Kuppler D-1 aus EP-A-0 287 833)

Weitere Schichtaufbauten 1 B bis 1 H wurden entsprechend hergestellt, die sich von Schichtaufbau 1A ausschließlich durch den in den Schichten 3, 5 und 8 verwendeten DIR-Kuppler unterscheiden.

In den Schichtaufbauten 1 B, 1 C und 1 D wurden folgende DIR-Kuppler (Vergleich) verwendet:

DIR-B (Schichtaufbau 1 B)

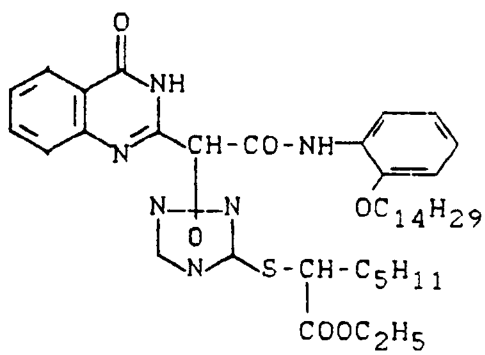

DIR-C (Schichtaufbau 1 C)

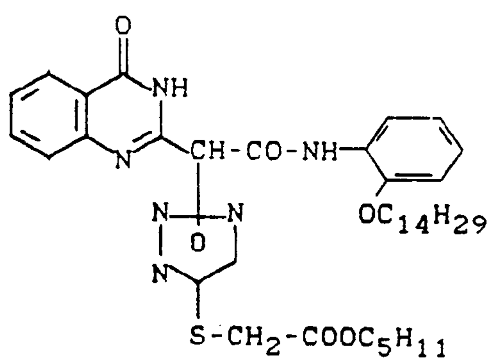

DIR-D (Schichtaufbau 1 D)

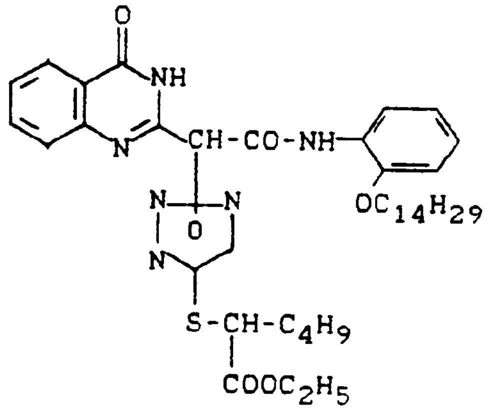

In den Schichtaufbauten 1 E bis 1 H wurden erfindungsgemäße DIR-Kuppler verwendet, die sich von den DIR-Kupplern DIR-A bis DIR-D ausschließlich dadurch unterscheiden, daß sie anstelle des Tetradecyloxyrestes einen o-Cyclohexylphenoxyrest tragen.

Die Entwicklung wurde nach Aufbelichtung eines Graukeils durchgeführt wie beschrieben in "The British Journal of Photography", 1974, Seiten 597 und 598.

Die Ergebnisse nach Verarbeitung sind in Tabelle 1 dargestellt. Die Interimageeffekte IIE berechnen sich wie folgt :

$$IIE_{bg} = \frac{\gamma_{rot} - \gamma_w}{\gamma_w} \; ; \; IIE_{pp} = \frac{\gamma_{grün} - \gamma_w}{\gamma_w} \; ;$$

$$IIE_{bg} = \frac{\gamma_{blau} - \gamma_w}{\gamma_w} \; ;$$

Dabei bedeutet:

$\gamma_{rot}$ Gradation bei selektiver Belichtung mit rotem Licht
$\gamma_{grün}$ Gradation bei selektiver Belichtung mit grünem Licht
$\gamma_{blau}$ Gradation bei selektiver Belichtung mit blauem Licht
$\gamma_w$ Gradation bei Belichtung mit weißem Licht

Der in Tabelle 1 angegebene Kanteneffekt KE ist die Differenz zwischen Mikro- und Makrodichte bei Makrodichte = 1, wie beschrieben in James, The Theory of the Photographic Process, 4th Edition, Macmillan Publishing Co. , Inc 1977, Seite 611. Dabei bedeutet:

$KE_{bg}$ KE in der rotsensibilisierten Schicht
$KE_{pp}$ KE in der grünsensibilisierten Schicht

Tabelle 1

| Schicht-aufbau | DIR- | DIR-Kuppler [mmol/100g $AgNO_3$] | $IIE_{gb}$ | $IIE_{pp}$ | $IIE_{bg}$ | $KE_{pp}$ | $KE_{bg}$ |
|---|---|---|---|---|---|---|---|
| 1 A | A | 5,0 | | 37 | 40 | 0,49 | 0,62 |
| 1 E | 1 | 5,0 | | 37 | 38 | - | - |
| 1 B | B | 2,7 | 0 | 24 | 32 | 0,20 | 0,31 |
| 1 F | 12 | 2,8 | 1 | 35 | 38 | 0,18 | 0,34 |
| 1 C | C | 3,1 | 5 | 37 | 18 | 0,15 | 0,17 |
| 1 G | 13 | 3,1 | 14 | 82 | 61 | 0,52 | 0,79 |
| 1 D | D | 2,9 | 12 | 36 | 33 | 0,33 | 0,40 |
| 1 H | 14 | 2,9 | 16 | 56 | 32 | 0,44 | 0,56 |

## Patentansprüche

1. Farbfotografisches Aufzeichnungsmaterial mit mindestens einer lichtempfindlichen Silberhalogenidemulsionsschicht und einem dieser zugeordneten DIR-Kuppler, dadurch gekennzeichnet, daß der DIR-Kuppler der folgenden allgemeinen Formel I entspricht

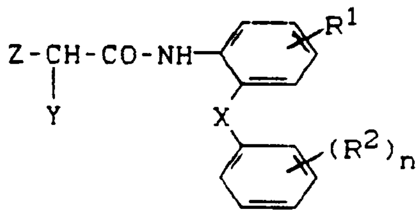

(I)

worin bedeuten

| | |
|---|---|
| $R^1$ | H, Cl, $-CF_3$, Alkoxy, Sulfamoyl; |
| $R^2$ | Cl, Alkyl, Cycloalkyl, Alkoxy, Alkoxycarbonyl, Carbamoyl; |
| n | 0, 1 oder 2; |
| X | O oder S; |
| Y | einen bei Farbentwicklung freisetzbaren Rest mit Silberhalogenidentwicklungsinhibierungsfunktion; |
| Z | Benzoyl, Pivaloyl, Carbamoyl oder einen Chinazolin-4-on-2-yl-Rest. |

## Claims

1. A color photographic recording material comprising at least one photosensitive silver halide emulsion layer and a DIR coupler associated therewith, characterized in that the DIR coupler corresponds to general formula I:

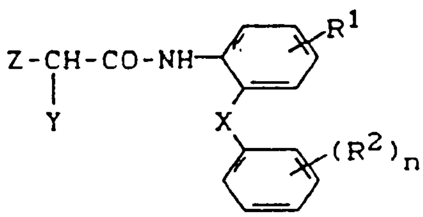

I

in which

| | |
|---|---|
| $R^1$ | is H, Cl, $-CF_3$, alkoxy, sulfamoyl; |
| $R^2$ | is Cl, alkyl, cycloalkyl, alkoxy, alkoxycarbonyl, carbamoyl; |
| n | is 0, 1 or 2; |
| X | is O or S; |
| Y | is a group with a silver halide development inhibiting function releasable during color development; |
| Z | is benzoyl, pivaloyl, carbamoyl or a quinazolin-4-on-2-yl group. |

## Revendications

1. Matériau pour enregistrement photographique en couleur ("produit photographique pour reproduction couleur") comportant au moins une couche d'émulsion d'halogénure d'argent photosensible et un coupleur DIR qui lui est associé, matériau caractérisé en ce que le coupleur DIR correspond à la formule générale I suivante :

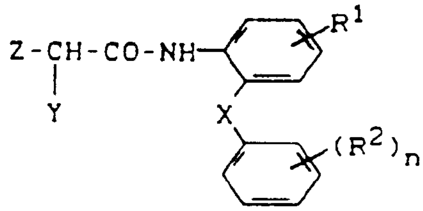

(I)

dans laquelle

- $R^1$ représente H, Cl, un groupe $-CF_3$, alcoxy, sulfamoyle;
- $R^2$ représente Cl, un groupe alkyle, cycloalkyle, alcoxy, alcoxycarbonyle, carbamoyle;
- n vaut 0, 1 ou 2;
- X représente O ou S;
- Y représente un reste libérable lors du développement de la couleur et ayant un rôle d'inhibition du développement de l'halogénure d'argent;
- Z représente un reste benzoyle, pivaloyle, carbamoyle ou un reste quinazoline-4-one-2-yle.